# EUROPEAN PATENT APPLICATION

(11) **EP 3 858 151 A1**
(43) Date of publication of application: **04.08.2021**
(21) Application number: 19864026.0
(22) Date of filing: 25.09.2019
(51) Int. Cl.: A23K 50/10, A23K 10/30, A61K 36/484, A61P 15/00

(54) **FEED ADDITIVE**

(30) Priority: 25.09.2018 JP 2018178878
(71) Applicant: Cokey Systems Co., Ltd., Matsusaka-shi, Mie 515-0041 (JP); Onishi, Takao, Ogoori-shi, Fukuoka, 838-0106 (JP)
(72) Inventor: AOYAGI Yoshito, Kato-gun, Hokkaido 080-0111 (JP); KOIWA Masateru, Ebetsu-shi, Hokkaido 069-0804 (JP); ONISHI Takao, Ogoori-shi, Fukuoka 838-0106 (JP)
(74) Representative: J A Kemp LLP
(86) International application number: PCT/JP2019/037524
(87) International publication number: WO 2020/067125

(57) **Abstract**

The present invention relates to an feed additive comprising a licorice extract for improving the quality of embryos obtained after the superovulation treatment, as well as, a method for improving the quality of the embryos obtained after the superovulation treatment comprising rearing to a mammal with superovulation treatment using a feed added with licorice extract.

## Description

### TECHNICAL FIELD

The present invention relates to a mammalian feed additive comprising licorice extract for improving the quality of embryos obtained after superovulation treatment, and a method for improving the quality of the embryos obtained after the superovulation treatment and for comprising rearing a mammal with superovulation treatment using a feed added with licorice extract to .

### BACKGROUND ART

A fertilized egg transplantation is widely carried out to efficiently promote the improvement and reproduction of mammals. Internally fertilized eggs are suitable for these fertilized eggs used for transplantation. Here, superovulation treatment is carried out to collect internally fertilized eggs from a fertilized female mammal. A superovulation treatment is carried out by gradually decreasing administration of follicle stimulating hormone to a female mammal, twice a day, for 3 to 5 days. However, depending on the physical condition and the like of the female mammal, a problem was reported that the frequency of developing a normal embryo from the fertilized egg obtained after this treatment may become low. In addition, in order to improving the rate of developing normal embryos from the fertilized eggs obtained by the superovulation treatment, a method of injecting a female hormone such as estradiol before treatment is known. However, a treatment such as an injection carried out by a veterinarian is a burden to dairy farmers, and thus, means performable by a breeder on his/her own has been sought.

### SUMMARY OF INVENTION

### Technical Problem

The problem of the present invention is to provide a mammalian feed additive or a method resulting in the improvement of embryo quality obtained after superovulation treatment.

### Means for Solving the Problems

The present inventors have carried out dedicated research to solve the above-mentioned problem, and as a result of this, they have discovered that the addition of a licorice extract to a mammalian feed results in the improvement of the quality of embryos obtained after the superovulation treatment of the mammals fed with the feed. Based on these findings, the present invention has been completed. That is, the present invention is as follows:
[1] A mammalian feed additive for improving the quality of embryos obtained after the superovulation treatment;
[2] the food additive according to [1], wherein the mammal is a cow;
[3] a method for improving the quality obtained after the superovulation treatment comprising rearing a mammal with superovulation treatment using a feed added with a licorice extract;
[4] the method according to [3], wherein the mammal is a cow;
[5] the method according to [4], wherein the licorice extract is administered at a dose of 20g/day/head, and
[6] the method according to [5], wherein the licorice extract is administered for 60 days or more.

### EFFECTS OF THE INVENTION

According to the present invention, the quality of the embryos obtained after the superovulation treatment can be improved.

### DESCRIPTION OF EMBODIMENTS

In the present invention, licorice refers to a plant which belongs to the genus of Glycyrrhiza. Licorice genus is a member of perennial plants of the family Fabaceae which naturally grows in the Mediterranean region, Asia Minor, Southern Russia, Central Asia, Northern China, North America, and the like. In the present invention, for example, the following may be used: Glycyrrhiza acanthocarpa, G. aspera, G. astragalina, G. bucharica, G. echinata (Russian licorice), G. eglandulosa, G. foetida, G. foetidissima, G. glabra (Spanish licorice), G. gontscharovii, G. iconica, G. inflate, G. korshinskyi, G. lepidota (American licorice), G. pallidiflora, G. squamulosa, G. triphylla, G. uralensis (Ural licorice), G. yunnanensis, G. inflata (Xinjiang licorice). One or two or more kinds of these could be used in combination.

The licorice extract used in the present invention can be used by appropriately processing the root and/or stem of the plant body. In the present invention, a commercially available product may be used, and moreover, those that were prepared by a known method in the present field may also be used. The content of the glycyrrhizic acid of the licorice extract used in the present invention may be 2.5% or more, preferably 5% or more, or more preferably 10% or more, and furthermore preferably, 13% or more.

The extract solvent of the licorice extract is not particularly limited; however, for example, water, alcohol (e.g., methanol, ethanol, n-propanol, and n-butanol), acetone, ethyl acetate, and the like, may be used. Furthermore, obtaining a dried powder of it, for example, the solvent of the extract is removed by methods ordinary carried out by a person skilled in the art, such as, reduced pressure drying and spray drying.

A superovulation treatment refers to, for example, a technique to induce to ovulate multiple ova using a hormone treatment which normally ovulates only one egg per time in a cow, and these multiple eggs are artificially inseminated to develop multiple fertilized eggs in the body, which are then collected by flushing the uterus with a liquid.

In the present invention, "improving the quality of the embryos obtained after the superovulation treatment" refers to, for example, the increase in the average number of transplantable embryos, the improvement of the average embryo quality score, and the increase in the rate of transplantable embryos.

In the present invention, mammals refer to, for example, monkeys, dogs, cats, goats, sheep, pigs, guinea pigs, rabbits, mice, rats, and humans, and preferably, it refers to cows.

In the present invention, "feed" is suitable for consumption by mammals, and the term refers to any compound, preparation, mixture, or composition used for the same intention. In the present invention, feed additive refers to an additive utilized for maintaining the quality of the feed or utilized as a supplementary nutrition, by adding to the cattle feed (feed) used in purpose of providing nutrition to cattle and the like.

One embodiment of the present invention is a method for improving the quality of embryos obtained after the superovulation treatment comprising rearing a mammal such as a cow with superovulation treatment using a feed added with licorice extract to.

The dose of the licorice extract of the present invention is not particularly limited as long as the effect is obtained. However, it is preferably 10-40g/day/head, preferably 15-25g/day/head, and more preferably, it is 20g/day/head.

The administration period of the licorice extract of the present invention may be any period as long as a desired effect is obtained; however, it is preferably administered for 60 days or more.

### EXAMPLES

As for the experimental animals, the Japanese Black (Kuroge Washu) (n=136) having 1-4 times parous and a normal uterus confirmed by ultrasound testing after at least 60 days from the last child delivery was used. To the individuals of the treatment group (n=90), 20g/day/head of licorice extract (Fabric Onishi, having at least 13% of glycyrrhizic acid content) was continuously fed for 60 to 90 days until the embryos were collected. The control group (n=46) did not consume any licorice from the last child delivery date to the collection of the embryos.

In both groups, a total amount of 20AU (Armour unit) of follicle stimulating hormone (FSH) was intramuscularly injected twice a day, in the morning and in the evening, for 3 consecutive days (diminishing dosage: 5AU x 2, 3AU x 2 and 2AU x 2) to cows with superovulation treatment 8 to 11 days after the onset of estrus. Twenty-five mg and 15 mg of ProstaglandinF_{2*α*} (Pronalgon F) were administered in the morning and evening of the 3rd day of FSH administration, respectively. Twelve hours and 24 hours after the onset of estrus, artificial insemination was carried out and the embryos were collected using uterine flushing method on day 7 after estrus.

The average number of the collected transplantable embryos/eggs and average embryo quality score (based on the IETS guidelines) in the treatment group and the control group were compared using the t-Test. Chi-squared test was used to compare the rate of transplantable embryos of these two groups.

No significant difference was observed between the treatment group (21.7±11.8) and the control group (15.8±11.3) with respect to the average number of eggs/embryos; however, the former tended to provide more oocytes. A significant difference was observed between the treatment group (13.1±8.3) and the control group (8.1±5.3) with respect to the average number of transplantable embryos (p,<0.05). Moreover, the average embryo quality score was significantly better in the treatment group (1.6±0.4) as compared to the control group (2.2±0.6). The rate of transplantable embryos was significantly better in the treatment group (60.3±26.4%) as compared to the control group (51.2±26.0%) (p<0.05).

These results show that feeding of licorice extracts to the Japanese black cows for 60 days or more resulted in the improvement of the quality of embryos obtained after the superovulation treatment and in the increase of the average number of transplantable embryos per head of cow.

### Industrial Applicability

The feed additive of the present invention is useful for improving the quality of the embryos obtained after the superovulation treatment.

## Claims

1. A mammalian feed additive comprising a licorice extract for improving the quality of embryos obtained after the superovulation treatment.

2. The feed additive according to claim 1, wherein the mammal is a cow.

3. A method for improving the quality of embryos obtained after the superovulation treatment comprising rearing the mammal with superovulation treatment using a feed added with a licorice extract.

4. The method according to claim 3, wherein the mammal is a cow.

5. The method according to claim 4, wherein the licorice extract is administered at a dose of 20g/day/head.

6. The method according to claim 5, wherein the licorice extract is administered for 60 days or more.
